Europäisches Patentamt

European Patent Office

Office européen des brevets

(19)

(11) Veröffentlichungsnummer: **0 044 915**
**A1**

(12) **EUROPÄISCHE PATENTANMELDUNG**

(21) Anmeldenummer: **81103947.8**

(22) Anmeldetag: **22.05.81**

(51) Int. Cl.³: **A 61 F 1/03**

(30) Priorität: **23.07.80 DE 3027832**

(43) Veröffentlichungstag der Anmeldung: **03.02.82**
**Patentblatt 82/5**

(84) Benannte Vertragsstaaten: **CH FR GB LI**

(71) Anmelder: **SIGRI ELEKTROGRAPHIT GMBH, Werner von Siemens-Strasse 18, D-8901 Meitingen (DE)**

(72) Erfinder: **Burri, Caius, Prof. Dr., Lärchenweg 12, D-7906 Herrlingen bei Ulm (DE)**
Erfinder: **Claes, Lutz, Dr., Kolpingstrasse 12, D-7914 Pfuhl bei Ulm (DE)**
Erfinder: **Neugebauer, Rainer, Dr., Liebigstrasse 8, D-7900 Ulm-Lehr (DE)**

(54) **Hüftendoprothese.**

(57) Schaftteil (2) einer kragenlosen Hüftgelenkendoprothese, der vorwiegend aus mit Kohlenstoffasern verstärktem Kohlenstoff besteht und dessen proximal-medialer Teil (3) einen elliptischen Querschnitt aufweist und im distalen Teil (4) zylindrisch ausgebildet ist. Die Verkeilung der Prothese erfolgt im Bereich des Adambogens, so daß bei Belastung der Prothese vergleichsweise kleine Biegemomente wirksam und übermäßige Beanspruchungen des Knochengewebes ausgeschlossen werden.

EP 0 044 915 A1

- 1 -

## Hüftendoprothese

Die Erfindung betrifft einen in den Femur einsetzbaren und mit diesem kraftschlüssig verbundenen Schaftteil einer Hüftendoprothese.

Als Ersatz für funktionsuntüchtig gewordene Gelenke, besonders auch der Hüftgelenke, sind Endoprothesen aus einer Vielzahl von Werkstoffen in verschiedenen Formen bekannt geworden, die in der Regel mit einem Knochenzement in einer Ausnehmung des Knochens, z.B. des Femurs, verankert sind. Der aus einem Polymeren bestehende Knochenzement führt häufig nicht zu befriedigenden Lösungen der Prothesenverankerung, da der Zement während der Aushärtung schwindet und somit keine ideale Verbindung von Knochen und Prothese bewirkt. Die Bioverträglichkeit wird zudem durch freigesetzte Monomere, Lösungsmittel und dergleichen begrenzt. Zur Überwindung dieser Schwierigkeiten sind mit dem Knochen verschraubbare Prothesen vorgeschlagen worden, welche ohne Knochenzement implantiert werden können. Bei einer solchen kraftschlüssigen vorgespannten Verbindung von Prothese und Knochen ist die Aufrechterhaltung der Vorspannung über längere Implantationszeiten problematisch, da Knochenumbauvorgänge an der Grenzfläche Knochen-Prothese zu einem Abfall der Vorspannkraft führen.

Ein Großteil der bekanntgewordenen Hüftendoprothesen weist zwischen dem stielartigen Schaft der Prothese und der Gelenkkugel eine wulstartige Auskragung auf, die auf der Resektionsfläche des Femurs aufliegt. Die Krafteinleitung ist bei dieser Form besonders ungünstig, da am medialen Teil des Kragens bevorzugt Druckspannungen auftreten. Die als Setzholz-Prothese bekanntgewordene Form weist keinen Kragen auf. Der Schaft ist keilförmig ausgebildet und verkeilt sich bei der Einführung in den ausgebohrten Markraum des Femurs kraftschlüssig mit dem Knochen. Die an die Toleranzen gestellten Anforderungen sind entsprechend wesentlich geringer als bei Prothesen mit Schraubgewinden. Nach den bekanntgewordenen Versuchsergebnissen ist die Standzeit von implantierten Setzholz-Prothesen recht günstig (Orthopäde 8, 75-78 (1979) ). Allerdings ist nicht auszuschließen, daß bei einer Verkeilung im distalen Bereich ein verhältnismäßig großes Spiel im proximalen Bereich verbleibt und auf die Verkeilungsstelle dann erhebliche schwellende Biegespannungen wirken. Eine Umbildung des Knochengewebes und die Lockerung der Prothese ist dann unvermeidlich.

Der Erfindung liegt die Aufgabe zugrunde, die möglichen Biegespannungen im Verankerungs- beziehungsweise Verkeilungsbereich einer kragenlosen Setzholz-Prothese zu verringern und damit für die Lebensdauer der Prothese schädliche Umbildungen des Knochengewebes in diesem Bereich auszuschließen oder wenigstens wesentlich zu verlangsamen.

Die Aufgabe wird mit einem kragenlosen Schaft gelöst, dessen gekrümmter proximal-medialer Teil einen elliptischen Querschnitt aufweist und dessen distaler Teil zylindrisch ausgebildet ist.

Beim Einführen des Prothesenschafts in den zylindrisch ausgebohrten Markraum, dessen Durchmesser geringfügig größer als der Durchmesser des zylindrisch ausgebildeten distalen Teils des Schafts ist, verkeilt sich der Schaft zwangsläufig am proximal-medialen Teil im Bereich des Adambogens. Der Hebelarm zwischen Gelenkkugel und Verankerungsstelle und damit das wirksame Biegemoment sind verhältnismäßig klein, so daß eine übermäßige Beanspruchung des Knochengewebes nicht möglich ist. Im distalen Bereich wird schließlich ein großflächiger Kontakt zwischen Prothese und Knochengewebe erreicht und damit eine sehr günstige Flächenpressung. Das Verhältnis der Längen von elliptischem und zylindrischem Teil der Prothese sollte etwa 1:2 bis 1:4 betragen. Der Prothesenschaft besteht bevorzugt aus Kohlenstoffasern enthaltendem Kohlenstoff, einem Werkstoff, der gegen lebende tierische und menschliche Gewebe inert ist und dessen Elastizitätsmodul durch Änderungen des Faseranteils und der Faserverteilung dem Modul des Knochens angepaßt werden kann. Durch diese als Isoelastizität bezeichnete Eigenschaft lassen sich Relativbewegungen zwischen Prothesenschaft und dem anliegenden Knochen weitgehend vermeiden.

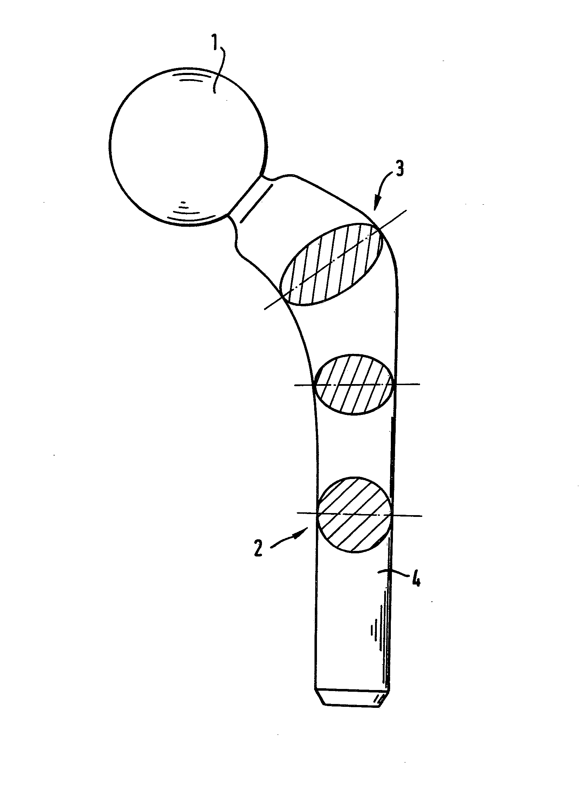

Die Erfindung wird im folgenden anhand einer Zeichnung beispielhaft erläutert. In der Figur ist eine Hüftendoprothese mit einer Gelenkkugel 1 und dem Schaftteil 2 dargestellt. Der gekrümmte proximal-mediale Teil des Schafts 3 weist einen elliptischen Querschnitt auf, der distale Teil des Schafts 4 ist zylindrisch ausgebildet.

Patentansprüche:

1. Schaftteil einer Hüftendoprothese,
   dadurch gekennzeichnet,
   daß der kragenlose, gekrümmte proximal-mediale Teil des
   Schafts einen elliptischen Querschnitt aufweist und der
   distale Teil des Schafts zylindrisch ausgebildet ist.

2. Schaftteil nach Patentanspruch 1,
   dadurch gekennzeichnet,
   daß das Verhältnis der Längen vom elliptischen und zylindrischen Schaftteil 1:2 bis 1:4 beträgt.

3. Schaftteil nach den Patentansprüchen 1 und 2, bestehend
   aus Kohlenstoffasern enthaltendem Kohlenstoff.

Europäisches
Patentamt

**EUROPÄISCHER RECHERCHENBERICHT**

00449.15
Nummer der Anmeldung
EP 81 10 3947

| | **EINSCHLÄGIGE DOKUMENTE** | | | KLASSIFIKATION DER ANMELDUNG (Int. Cl.³) |
|---|---|---|---|---|
| Kategorie | Kennzeichnung des Dokuments mit Angabe, soweit erforderlich, der maßgeblichen Teile | | betrifft Anspruch | A 61 F 1/03 |
| | FR - A - 2 404 429 (BREARD)<br>* Anspruch 1; Abbildungen * | | 1 | |
| | US - A - 4 153 953 (GROBBELAAR)<br>* Spalte 3, Zeilen 6-25; Abbildungen * | | 1 | |
| | DE - A - 2 621 123 (BODER)<br>* Anspruch 1 * | | 3 | **RECHERCHIERTE SACHGEBIETE (Int. Cl.³)** |
| A | MEDICAL PROGRESS TECHNOLOGY, Band 5, Nr. 2, September 1977, Seiten 73-101<br>P.J. BROCKHURST et al.: "Design of total hip prosthesis"<br>* Seite 80 * | | 1 | A 61 F |

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 29-10-1981 | STEENBAKKER |

EPA form 1503.1 06.78